# EUROPÄISCHE PATENTSCHRIFT

(11) **EP 0 941 404 B1**
(45) Veröffentlichungstag und Bekanntmachung des Hinweises auf die Patenterteilung: **10.11.2004**
(21) Anmeldenummer: 98913735.1
(22) Anmeldetag: 26.03.1998
(51) Int. Cl.: F04B 43/067

(54) **PUMP- UND DOSIERVORRICHTUNG**
PUMPING AND METERING DEVICE
DISPOSITIF DE POMPAGE ET DE DOSAGE

(30) Priorität: 26.09.1997 DE 19742632
(43) Veröffentlichungstag der Anmeldung: 15.09.1999
(73) Patentinhaber: Fresenius Medical Care Deutschland GmbH, 61350 Bad Homburg v.d.H. (DE)
(72) Erfinder: HERKLOTZ, Martin, D-64347 Griesheim (DE); SCHNEIDER, Hans-Peter, D-61267 Neu-Anspach (DE); BIGALKE, Jörg, D-60327 Frankfurt (DE); DÖNIG, Rainer, D-60489 Frankfurt (DE); HÄCKER, Jürgen, D-74189 Weinsberg (DE)
(74) Vertreter: Laufhütte, Dieter, Dr.-Ing.
(86) Internationale Anmeldenummer: PCT/EP1998/001792
(87) Internationale Veröffentlichungsnummer: WO 1999/017019

(56) Entgegenhaltungen:
- EP-A- 0 641 935
- DE-A- 2 838 177
- DE-U- 29 608 950
- US-A- 3 771 694
- US-A- 4 303 376
- US-A- 4 468 222
- US-A- 5 056 036

## Beschreibung

Die Erfindung betrifft eine Pumpvorrichtung zur Förderung, Bilanzierung und Dosierung von Flüssigkeiten, insbesondere von medizinischen Flüssigkeiten, wie z.B. Blut oder Dialyseflüssigkeiten, mit einer eine Antriebsvorrichtung und einen Kolben umfassenden Kolbeneinheit sowie einer Membraneinheit, die eine Membran sowie eine erste Kammer umfaßt, die durch die Membran begrenzt wird. Die Pumpvorrichtung umfaßt ferner eine Hydraulikeinheit, die einen Raum zur Aufnahme einer Hydraulikflüssigkeit aufweist, der mit dem Kolben der Kolbeneinheit und mit der ersten Kammer der Membraneinheit in Verbindung steht. Solche Vorrichtungen sind bekannt aus den Dokumenten US-A-4 468 222, US-A-5 056 036 und DE 28 38 177 A.

Ein wesentlicher Vorteil eines derartigen Pumpsystems besteht darin, daß die Vorteile von Kolbenmaschinen mit denen von Membraneinheiten kombinierbar sind. Die als Kolbenpumpe arbeitende Kolbeneinheit dient als interne Verdrängerpumpe, die mittels einer Hydraulikflüssigkeit mit der Membran der Membraneinheit in Verbindung steht. Die Hydraulikflüssigkeit befindet sich in einem geschlossenen System und überträgt die Axialbewegung des Kolbens auf die Membran, die dabei entsprechend in der Membraneinheit bewegt wird.

Die exakte Dosierung von Flüssigkeiten ist beispielsweise im Bereich der Dialyse von Bedeutung, bei der Flüssigkeiten bekannter Zusammensetzung mit genau bestimmbaren Raten gefördert werden müssen. Die dabei verwendeten Dialyseflüssigkeiten sind aus einer Vielzahl von Substanzen zusammengesetzt, deren Art und Menge auf die Bedürfnisse einer adäquaten und individuell abgestimmten Patientenbehandlung auszurichten sind. Die wesentlichen Aufgaben einer Dialysevorrichtung sind die Förderung mit exakt vorgebbaren Dosierraten sowie die quantitative Fassung der geförderten Mengen zum Zwecke der Bilanzierung. Bei bekannten Dialysesystemen ist es von Nachteil, daß diese Aufgaben von unterschiedlichen Einheiten durchgeführt werden, was in entsprechend komplexen, schwergewichtigen und schlecht handhabbaren Geräten resultiert.

Ein kompaktes Dosiersystem ist beispielsweise aus der Patentschrift EP 0 376 497 aus dem Bereich der Beschichtung von Halbleiterbauteilen bekannt, bei der ebenfalls eine präzise Förderung und Dosierung flüssiger Medien erforderlich ist. Hier wird eine gattungsgemäße Pumpvorrichtung beschrieben, bei der eine Membran auf ihrer einen Seite mit einer in einem entsprechenden Raum befindlichen Hydraulikflüssigkeit in Kontakt steht. Die gewünschte Bewegung der Membran erfolgt mittels einer Kolbeneinheit, wobei die Bewegung eines Kolbens der Kolbeneinheit durch die Hydraulikflüssigkeit auf die Membran übertragen wird. Die Steuerung dieser Dosiereinheit erfolgt auf der Basis der Anzahl von Impulsen pro Zeiteinheit, die auf den den Kolben antreibenden Motor der Vorrichtung aufgegeben werden. Die Relation zwischen der Pulszahl pro Zeiteinheit und der gewünschten Förderhöhe der Pumpvorrichtung wird durch Kalibrierung vor dem Einsatz bestimmt und im Betrieb zur Steuerung der Pumpvorrichtung herangezogen. Ein Nachteil einer derartigen Vorgehensweise besteht darin, daß es beispielsweise durch eine mangelhafte Bestimmung der erforderlichen Pulszahl, durch wechselnde Belastungen oder durch eine ungenügende Kalibrierung zu einer fehlerhaften Beziehung zwischen Pulszahl pro Zeiteinheit und Förderhöhe kommen kann, die eine exakte Dosierung erschwert.

Es ist die Aufgabe der vorliegenden Erfindung, eine Pumpvorrichtung dahingehend weiterzubilden, daß die Zuverlässigkeit der Dosierung und Bilanzierung erhöht wird.

Diese Aufgabe wird ausgehend von einer gattungsgemäßen Pumpvorrichtung dadurch gelöst, daß eine Regeleinheit zur Regelung einer vorgebbaren Dosierrate und/oder -menge sowie eine Meßvorrichtung vorgesehen ist, durch die unmittelbar die axiale Position des Kolbens der Kolbeneinheit bestimmbar ist und die mit der Regeleinheit verbindbar ist. Auf diese Weise wird eine unmittelbare und zuverlässige Bestimmung der Kolbenposition sowie nach Berücksichtigung der Zeit deren zeitliche Änderung möglich, wobei die Bestimmung von der Funktion und Zuverlässigkeit der Antriebseinheit unabhängig ist. Neben diesen Funktionen der Förderung und Dosierung erfüllt die erfindungsgemäße Vorrichtung ferner die Aufgabe, daß die Menge der geförderten Fluide quantitativ erfaßbar ist, wodurch eine Bilanzierung, beispielsweise während einer Dialysebehandlung, möglich wird. Somit wird ein kompaktes und zuverlässiges Förder-, Dosier- und Bilanziersystem geschaffen, welches neben Platz- und Gewichtsvorteilen zusätzlich eine günstige Herstellung und Wartung ermöglicht. Insbesondere eignet sich das erfindungsgemäße System für die Heimdialyse, da bei geringen Abmessungen ein Leistungsspektrum geschaffen wird, das den Einsatz von weiteren Kontroll- und Überwachungsvorrichtungen, die einen Aufenthalt des Patienten in einer Klinik erfordern würden, überflüssig macht. Weitere Anwendungsgebiete der erfindungsgemäßen Pumpvorrichtung sind die Peritonealdialyse, die Hämofiltration sowie verwandte Verfahren. Aufgrund der unmittelbar bestimmbaren Position des Kolbens sowie der daraus ermittelbaren Kolbengeschwindigkeit ermöglicht die erfindungsgemäße Pumpvorrichtung unter Berücksichtigung der Durchschnittsfläche des Kolbens die Berechnung sämtlicher notwendiger Systemdaten, wie z.B. des geförderten Volumens oder der Förderrate. Neben den genannten kinematischen Parametern können durch die Verwendung eines Hydrauliksensors auch zusätzlich die Druckverhältnisse überwacht und reguliert werden. Außerdem können Teilvolumina erkannt und eingestellt werden.

Besonders vorteilhaft ist es, wenn der Kolben einen Kolbenkopf zur Förderung der Hydraulikflüssigkeit sowie einen Kolbenschaft aufweist und die Meßvorrichtung derart angeordnet ist, daß die axiale Position des Kolbenschaftes bestimmbar ist.

Die erfindungsgemäße Meßvorrichtung, mit der unmittelbar die axiale Position des Kolbens bzw. des Kolbenschaftes ermittelbar ist, kann optische, elektromechanische und/oder elektrische Sensoren aufweisen.

Gemäß einer bevorzugten Ausgestaltung kann eine zweite Kammer durch ein an der Membraneinheit lösbar angebrachter Membranpumpkopf gebildet werden, wobei die zweite Kammer auf der der ersten Kammer gegenüberliegenden Membranseite angeordnet ist und wobei der Membranpumpkopf wenigstens einen Einlaß und wenigstens einen Auslaß aufweist. Dabei dient die zweite Kammer als Förderkammer, die das zu fördernde Medium aufnimmt, während die erste Kammer mit Hydraulikflüssigkeit beaufschlagt wird, um eine entsprechende Bewegung der Membran zu bewirken.

Im montierten Zustand des Membranpumpkopfes kann die zweite Kammer unmittelbar an die Membran der Membraneinheit angrenzen. In diesem Fall wird die Membran von beiden Seiten mit Flüssigkeit beaufschlagt, wobei auf der einen Membranseite die Hydraulikflüssigkeit und auf der anderen Membranseite die zu fördernde Flüssigkeit vorliegt.

Besonders vorteilhaft ist es, wenn die zweite Kammer von einer Membran begrenzt wird, die im montierten Zustand des Membranpumpkopfes an die Membran der Membraneinheit angrenzt. In diesem Fall liegen die beiden Membranen aneinander an, wobei die Bewegung der Hydraulikflüssigkeit zunächst die Membran der Membraneinheit in Bewegung versetzt, und durch die Berührung dieser Membran mit der Membran des Kopfstückes die zu fördernde Flüssigkeit entsprechend in die zweite Kammer eingeführt oder aus dieser abgegeben wird. Eine derartige Ausgestaltung der erfindungsgemäßen Pumpvorrichtung ist insbesondere deshalb von Vorteil, da in diesem Fall zwei vollständig voneinander getrennte Systeme vorliegen. Während die erfindungsgemäße Pumpvorrichtung beispielsweise gemäß Anspruch 1 die Fördereinheit darstellt, dient der Membranpumpkopf mit Membran zum Abschluß des zu fördernden Mediums und zur Stofftrennung mit der erfindungsgemäßen Vorrichtung. Die Stofftrennung führt nicht nur dazu, daß das Hydraulikfluid sowie das zu fördernde Medium nicht verunreinigt werden, sondern auch dazu, daß die Teile der erfindungsgemäßen Pumpvorrichtung durch das zu fördernde Medium weder angegriffen noch verunreinigt werden. In diesem Fall richtet sich die Wahl des Membranmaterials der Membraneinheit nicht vorwiegend nach dem Korrosionsverhalten, sondern in erster Linie nach dem Kriterium der Langzeitstabilität.

In weiterer Ausgestaltung der Erfindung ist vorgesehen, daß der Einlaß und/oder der Auslaß des Kopfstückes absperrbar ist. Hierzu werden insbesondere Ventile oder Klemmen vorgesehen. Diese haben beispielsweise die Aufgabe, beim Einsaugen von zu förderndem Medium in die zweite Kammer die Absperrung des Auslasses des Kopfstückes zu bewirken, während beim Ausstoßen des angesaugten Mediums das Einlaßventil geschlossen und das Auslaßventil entsprechend geöffnet wird. Dabei ist es nicht erforderlich, daß beim Ausstoßvorgang die gesamte in der zweiten Kammer befindliche Flüssigkeit abgegeben wird. Vielmehr ist es möglich, daß nur eine der maximal möglichen Kolbenposition entsprechende Menge an angesaugter Flüssigkeit abgeführt wird.

Besonders vorteilhaft ist es, wenn das Kopfstück derart ausgeführt ist, daß es für den Einmalgebrauch verwendbar ist. Während die erfindungsgemäße Pumpvorrichtung zur exakten Bewegung der Membran und somit zur Dosierung dient, erfüllt das Kopfstück die Aufgabe, die eigentliche Förderung der Flüssigkeit, durchzuführen. Das austauschbare und gemäß der vorliegenden Ausgestaltung als Wegwerfartikel ausgeführte Kopfstück, das an die Pumpvorrichtung montiert werden kann, weist den Vorteil auf, daß die Reinigung schwer zugänglicher Komponenten, wie z.B. Ventile, nicht nötig ist, da das Kopfstück nach einer erfolgten Benutzung nicht erneut eingesetzt wird. Somit bildet die Membran der Membraneinheit die Schnittstelle der Pumpvorrichtung mit dem als Einmalartikel ausgefüllten Kopfstück, in dem der Stofftransport bzw. die Medienförderung vom oder zum Patienten stattfinden soll. Die eindeutige Medientrennung, die einen direkten Kontakt von Maschinensystemteilen der erfindungsgemäßen Pumpvorrichtung mit dem zu fördernden Medium verhindern, bewirkt, daß weder Verunreinigungen in die Dialyseflüssigkeit gelangen können, noch daß durch Leckagen Dialyseflüssigkeit beispielsweise in die erfindungsgemäße Pumpvorrichtung übertreten kann. Ein weiterer Vorteil besteht darin, daß die Dosier- und Bilanziergenauigkeit der Pumpvorrichtung unabhängig von der Formgenauigkeit des als Wegwerfartikel ausgeführten Kopfstückes ist, da sämtliche für die Bilanzierung und Dosierung notwendigen Komponenten in der Kolbenpumpvorrichtung und nicht in dem montierbaren Membranpumpkopf vorgesehen sind.

In weiterer Ausgestaltung der Erfindung ist vorgesehen, daß ein Druckaufnehmer vorhanden ist, der mit dem Raum der Hydraulikeinheit in Verbindung steht. Durch die Verwendung eines derartigen Druckaufnehmers in der Hydraulikeinheit kann eine individuell einstellbare Förderdruckbegrenzung bzw. -anzeige des Systems erreicht werden. Dies ist insbesondere dann von Bedeutung, wenn beispielsweise das Auslaßventil des Kopfstückes versehentlich nicht öffnet.

Besonders vorteilhaft ist es, wenn der Druckaufnehmer mit dem Antrieb der Kolbeneinheit verbindbar ist. Auf diese Weise ist es möglich, daß der Drucksensor bei Erreichen eines Grenzwertes den Pumpenantrieb unterbricht, um die in das System eingeleiteten Kräfte zu begrenzen.

In weiterer Ausgestaltung der Erfindung ist vorgesehen, daß die Antriebsvorrichtung der Kolbeneinheit einen Linearantrieb umfaßt. Als Linearantriebe kommen beispielsweise Exzenter-, Spindelstangen- und Zahnstangenantriebe sowie Pneumatikkolben mit Kompressorantrieben in Frage.

Das Hydrauliksystem der erfindungsgemäßen Pumpvorrichtung kann ein Entlüftungsventil aufweisen, wodurch gewährleistet wird, daß die Hydraulikflüssigkeit frei von Gasen ist. Dies ist deshalb von besonderer Bedeutung, da die Abstimmung der Bewegungen der Kolbeneinheit und der Membran nur dann exakt erfolgen kann, wenn das Übertragungsmedium inkompressibel ist, wie dies z.B. bei gasfreien Flüssigkeiten der Fall ist.

In weiterer Ausgestaltung der Erfindung ist vorgesehen, daß eine Recheneinheit vorhanden ist, die mit der Meßvorrichtung und/oder mit der Regeleinheit verbunden ist und mittels derer eine Bilanzierung der geförderten Medien durchführbar ist. Aufgrund der unmittelbaren Bestimmung der Kolbenposition bzw. der Ermittlung der zeitlichen Änderung ist die Erfassung der bisher geförderten Medien, die für eine exakte Überwachung des Prozesses erforderlich ist, möglich.

Die Recheneinheit kann dabei in der Regeleinheit integriert sein.

Um die Handhabbarkeit der erfindungsgemäßen Pumpvorrichtung zu verbessern, kann die Kolbeneinheit auf einem Chassis angeordnet sein.

Besonders vorteilhaft ist es, wenn die Membran der Membraneinheit zwei aus einem nicht dehnbaren Material bestehende Membranlagen sowie einen sich zwischen beiden Membranlagen erstreckenden und mit einem inkompressiblen Medium derart gefüllten Zwischenraum umfaßt, daß die Membranlagen eine bezüglich des Zwischenraumes nach außen gerichtete Wölbung aufweisen. Durch eine derartige Ausgestaltung wird der den vorbekannten elastischen Membranen anhaftende Nachteil vermieden, daß sich in Abhängigkeit und infolge der zwischen beiden Seiten der Membran anliegenden Druckdifferenz eine unerwünschte Verformung bzw. Auslenkung der Membran einstellt. Diese Auslenkung hat zur Folge, daß eine exakte Korrelation zwischen der Position des Kolbens der Kolbeneinheit und der Membranauslenkung und somit eine genaue Dosierung außer bei stets konstanten Druckverhältnissen nicht möglich ist. Demgegenüber wird die erfindungsgemäße Membran unabhängig von den Druckverhältnissen stets in einer eindeutigen Position gehalten, wodurch eine reproduzierbare Korrelation zwischen Kolbenbewegung und Fördermenge sichergestellt wird.

In weiterer Ausgestaltung der vorliegenden Erfindung ist vorgesehen, daß die Membranlagen-durch ein Distanzstück voneinander getrennt angeordnet sind. Dadurch wird bei entsprechender Ausgestaltung des Distanzstückes insbesondere die Befüllung des zwischen den Membranlagen befindlichen Zwischenraumes erleichtert.

Weitere Einzelheiten und Vorteile der erfindungsgemäßen Pumpvorrichtung werden aus der Zeichnung ersichtlich. Es zeigen:
- Fig. 1:: eine Prinzipskizze der erfindungsgemäßen Pumpvorrichtung,
- Fig.2:: eine perspektivische Ansicht der erfindungsgemäßen Pumpvorrichtung mit Kolben- und Membraneinheit und
- Fig. 3:: eine Schnittdarstellung durch eine erfindungsgemäße Pumpvorrichtung mit einer eine zweilagige Membran aufweisenden Membraneinheit.

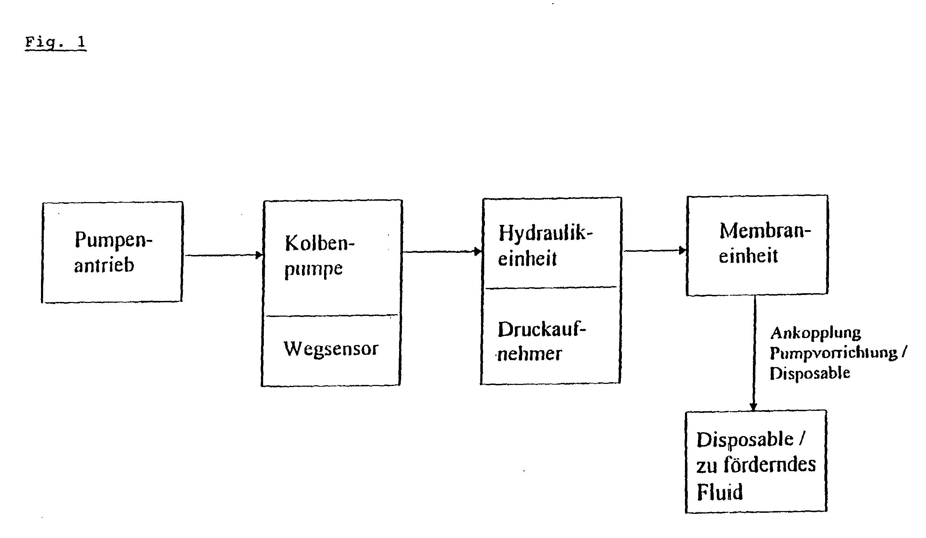

Wie aus Fig. 1 hervorgeht, treibt ein Pumpenantrieb, der beispielsweise einen Linearantrieb umfaßt, den Kolben einer als Kolbenpumpe dargestellten Einheit an. Dabei ermittelt die erfindungsgemäße Meßvorrichtung, die gemäß dem Ausführungsbeispiel in Fig. 1 als Wegsensor dargestellt ist, die exakte Position des Kolbens.

Der Einsatz einer Kolbenpumpe als Dosiereinheit erfolgt aufgrund der Tatsache, daß diese bevorzugt als Dosierpumpen einsetzbar sind, da nicht nur eine exakte Dosierung möglich ist, sondern auch eine einfache und flexible Einstellung auf neue Sollwerte erfolgen kann.

Die Hydraulikeinheit überträgt die Bewegung der Kolbenpumpe auf die Membraneinheit. Die Hydraulikeinheit weist einen Druckaufnehmer auf, der im Falle des Erreichens eines vorgebbaren Druckgrenzwertes beispielsweise ein Alarmsignal abgibt oder unmittelbar den Antrieb der Kolbeneinheit unterbricht.

Gemäß dem vorliegenden Ausführungsbeispiel ist an die Membraneinheit ein als "Disposable" gekennzeichnetes Kopfstück fixierbar, das mit dem zu fördernden Fluid in Verbindung steht. Ein direkter Kontakt dieses Fluids mit den Bestandteilen der erfindungsgemäßen Pumpvorrichtung kann somit wirksam verhindert werden.

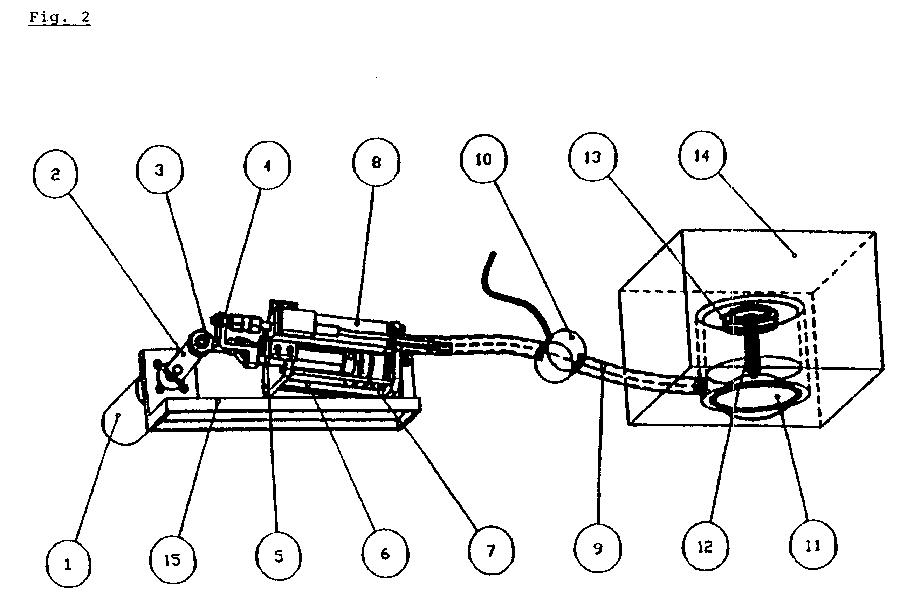

Fig. 2 zeigt eine perspektivische Ansicht der erfindungsgemäßen Pumpvorrichtung mit der als Elektro-Getriebemotor ausgeführten Antriebsvorrichtung 1. Der Exzenterhebel 2 überträgt die Rotation des Getriebemotors mittels des Kugelgelenkkopfes 3 auf den Kolben 7.

Die Kolbenstange des Kolbens 7 weist eine Verdrehsicherung 5 auf, die an einem Flansch 4 befestigt ist. An dem Flansch 4 ist ferner die als Wegsensor ausgeführte Meßvorrichtung 8 angeordnet.

Der als Präzisionskolben ausgeführte Kolben 7 mit Kolbenstange läuft in einem Präzisionszylinder 6 mit Pumpendeckel und Zuganker.

Die Bewegungen des Kolbens 7 werden durch die Hydraulikeinheit 9, die im vorliegenden Ausführungsbeispiel als Leitung ausgeführt ist, auf die Membraneinheit 14 übertragen. Diese weist die Membran 11 auf, die die erste Kammer 13 begrenzt. Die erste Kammer 13 enthält gemäß dem vorliegenden Ausführungsbeispiel das Entlüftungsventil 12. Sammeln sich in der Hydraulikeinheit 9 Gase an, so werden diese mittels des Entlüftungsventils 12 aus der Hydraulikeinheit entfernt, damit stets ein inkompressibles Übertragungsmedium zwischen Kolbeneinheit und Membraneinheit zur Verfügung steht.

Der Druckaufnehmer 10, der sich in der Hydraulikeinheit 9 befindet, die mit Hydraulikflüssigkeit gefüllt ist, ist mit einer Anzeige und/oder der Antriebsvorrichtung 1 über einen Rechner verbunden und schaltet diese im Falle des Erreichens oder Überschreitens eines vorgebbaren Grenzwertes ab. Somit wird auf sichere Weise beispielsweise bei einer Blockade der Hydraulikeinheit 9 oder einen Defekt der Ventile oder Klemmen des Kopfstückes die maximale Druckhöhe im gesamten System begrenzt.

Um die Handhabbarkeit der erfindungsgemäßen Pumpvorrichtung zu verbessern ist die Kolbeneinheit mit Antriebsvorrichtung 1 auf einem Chassis 15 befestigt.

Auf die Membraneinheit 14 wird in nicht dargestellter Weise ein als Einmalartikel ausgeführter Membranpumpkopf aufgesetzt und bildet somit die zweite Kammer, die beispielsweise durch die Membran 11 der Membraneinheit 14 begrenzt wird.

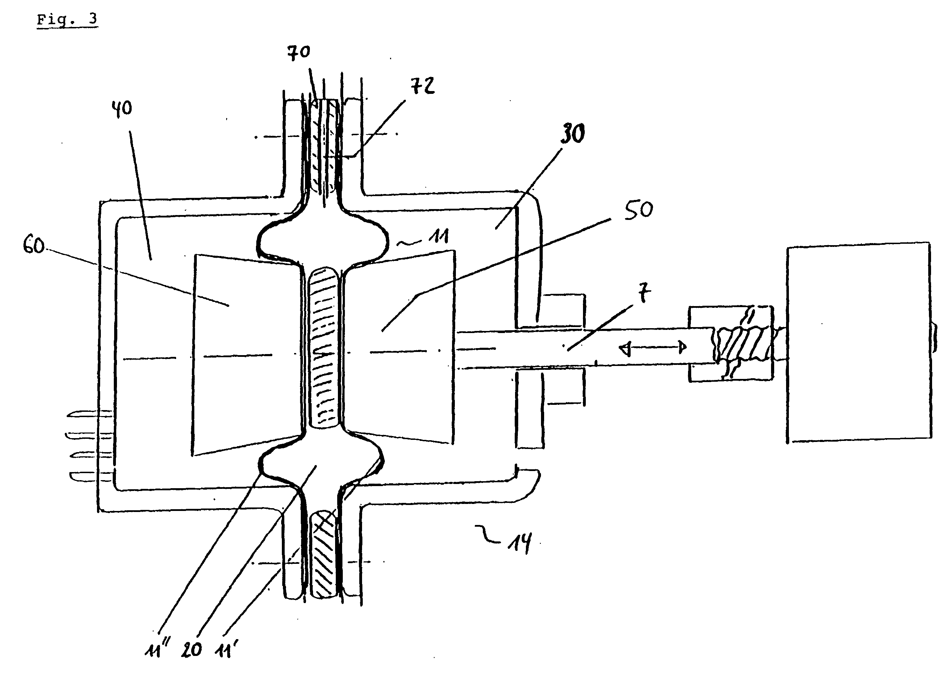

Fig. 3 zeigt eine Schnittdarstellung durch eine erfindungsgemäße Pumpvorrichtung mit einer eine zweilagige Membran 11 aufweisenden Membraneinheit 14. Die Membran 11 besteht aus den Membranlagen 11' und 11", die den Zwischenraum 20 begrenzen. Beide Membranlagen 11', 11" bestehen aus einem nicht dehnbaren, beispielsweise gewebeverstärkten, Material. Der Zwischenraum 20 ist mit einem inkompressiblen Medium, wie z.B. Öl, gefüllt.

Die Volumina der ersten Kammer 30 sowie der auf der gegenüberliegenden Seite der Membran 11 angeordneten Kammer 40 bleiben unabhängig von dem zwischen beiden Kammern 30, 40 herrschenden Druckgefälle konstant. Daraus ergibt sich, daß die bei bekannten Membranen auftretenden Auslenkungen, die bei einer Druckdifferenz über der Membran auftreten und somit zu einer fehlerhaften Beziehung zwischen Kolbenposition und Membranauslenkung führen, vermieden werden können.

Eine Bewegung des Kolbens 7 der Kolbeneinheit führt erfindungsgemäß zu einer Verschiebung des Zwischenraums 20, ohne daß dabei dessen Volumen verändert wird. Eine Volumenänderung in der Kammer 40 wird ausschließlich durch die Bewegung des Kolbens 7 bzw. des daran angeordneten Endstückes 50 bewirkt. Auf der der Kammer 40 zugewandten Seite der Membran 11 ist zur Fixierung der Membranlage 11" die Abstützung 60 vorgesehen.

Die Membranlagen 11', 11" sind durch das Distanzstück 70 voneinander getrennt angeordnet. Das Distanzstück 70 weist eine Öffnung 72 auf, die dazu dient, den Zwischenraum 20 mit einem geeigneten inkompressiblen Medium zu befüllen.

Die erfindungsgemäße Pumpvorrichtung stellt ein Pump-, Bilanzier- und Dosiersystem für medizinische Fluide wie Blut und Dialyseflüssigkeiten dar, das zuverlässig arbeitet und leicht handhabbar ist. Sie ist vorteilhaft insbesondere im Bereich der Peritonealdialyse, der Hämodialyse, der Hämofiltration und verwandter Verfahren einsetzbar. Durch die Kombination und Kopplung einer Kolbeneinheit mit Meßvorrichtung sowie einer Membraneinheit mittels einer Hydraulikeinheit werden die guten Eigenschaften von Kolbensystemen, die sich insbesondere auf die exakte Dosierung erstrecken, mit den Vorteilen einer Membraneinheit, die eine sichere Trennung von Arbeits- und Fördermedien ermöglicht, gewährleistet, wobei die erfindungsgemäße Meßvorrichtung eine exakte Dosierung und Bilanzierung ermöglicht.

## Patentansprüche

1. Pumpvorrichtung zur Förderung und Dosierung insbesondere medizinischer Flüssigkeiten mit
einer eine Antriebsvorrichtung (1) und einen Kolben (7) umfassenden Kolbeneinheit,
einer Membraneinheit (14), die mindestens eine Membran (11) sowie eine erste Kammer (13) umfaßt, die durch die Membran (11) begrenzt wird, sowie
einer Hydraulikeinheit (9), die einen Raum zur Aufnahme einer Hydraulikflüssigkeit aufweist, der mit dem Kolben (7) der Kolbeneinheit und der ersten Kammer (13) der Membraneinheit (14) in Verbindung steht,
wobei eine Regeleinheit zur Regelung einer vorgebbaren Dosierrate und/oder Menge sowie eine Messvorrichtung (8) vorgesehen ist, durch die unmittelbar die axiale Position des Kolbens (7) der Kolbeneinheit bestimmbar ist und die mit der Regeleinheit verbindbar ist,
**dadurch gekennzeichnet,**
**dass** mit der Membraneinheit (14) lösbar ein Kopfstück derart verbindbar ist, dass eine auf der der ersten Kammer (13) gegenüberliegende Membranseite angeordnete zweite Kammer gebildet wird, wobei der Membranpumpkopf wenigstens einen Einlass und wenigstens einen Auslass aufweist und dass die Membranlagen (11', 11") durch ein Distanzstück (70) voneinander getrennt angeordnet sind.

2. Pumpvorrichtung nach Anspruch 1, **dadurch gekennzeichnet, daß** der Kolben (7) einen Kolbenkopf und einen Kolbenschaft aufweist und die Meßvorrichtung (8) derart angeordnet ist, daß die axiale Position des Kolbenschaftes bestimmbar ist.

3. Pumpvorrichtung nach Anspruch 1 oder 2, **dadurch gekennzeichnet, daß** die Meßvorrichtung (8) optische und/oder elektronische Sensoren aufweist.

4. Pumpvorrichtung nach einem der Ansprüche 1 bis 3, **dadurch gekennzeichnet, daß** im montierten Zustand des Membranpumpkopfes die zweite Kammer unmittelbar an die Membran (11) der Membraneinheit (14) angrenzt.

5. Pumpvorrichtung nach einem der Ansprüche 1 bis 3, **dadurch gekennzeichnet, daß** die zweite Kammer von einer Membran begrenzt wird, die im montierten Zustand des Membranpumpkopfes an die Membran (11) der Membraneinheit (14) angrenzt.

6. Pumpvorrichtung nach einem der vorherigen Ansprüche, **dadurch gekennzeichnet, daß** die Membranen luftfrei aufeinanderliegen.

7. Pumpvorrichtung nach einem oder mehreren der Ansprüche 1 bis 5, **dadurch gekennzeichnet, daß** der Einlaß und/oder der Auslaß des Membranpumpkopfes absperrbar ist.

8. Pumpvorrichtung nach einem oder mehreren der Ansprüche 1 bis 7, **dadurch gekennzeichnet, daß** das Kopfstück derart ausgeführt ist, daß es für den Einmalgebrauch verwendbar ist.

9. Pumpvorrichtung nach einem oder mehreren der Ansprüche 1 bis 8, **dadurch gekennzeichnet, daß** ein Druckaufnehmer (10) vorgesehen ist, der mit dem Raum der Hydraulikeinheit in Verbindung steht.

10. Pumpvorrichtung nach Anspruch 9, **dadurch gekennzeichnet, daß** der Druckaufnehmer (10) über einen Rechner oder Motorcontroller mit der Antriebsvorrichtung (1) der Kolbeneinheit verbindbar ist.

11. Pumpvorrichtung nach einem oder mehreren der Ansprüche 1 bis 10, **dadurch gekennzeichnet, daß** die Antriebsvorrichtung (1) der Kolbeneinheit einen Linearantrieb umfaßt.

12. Pumpvorrichtung nach Anspruch 11, **dadurch gekennzeichnet, daß** der Linearantrieb durch eine Zahnstange gebildet ist.

13. Pumpvorrichtung nach einem oder mehreren der Ansprüche 1 bis 12, **dadurch gekennzeichnet, daß** die Hydraulikeinheit ein Entlüftungsventil (12) aufweist.

14. Pumpvorrichtung nach einem oder mehreren der Ansprüche 1 bis 13, **dadurch gekennzeichnet, daß** eine Recheneinheit vorgesehen ist, die mit der Meßvorrichtung (8) und/oder mit der Regeleinheit verbunden ist und mittels derer eine Dosierung; Flußrateneinstellung und damit auch eine Bilanzierung der geförderten Medien durchführbar ist.

15. Pumpvorrichtung nach Anspruch 14, **dadurch gekennzeichnet, daß** die Recheneinheit in der Regeleinheit integriert ist.

16. Pumpvorrichtung nach einem oder mehreren der Ansprüche 1 bis 15, **dadurch gekennzeichnet, daß** die Kolbeneinheit auf einem Chassis (15) angeordnet ist.

17. Pumpvorrichtung nach einem oder mehreren der Ansprüche 1 bis 16, **dadurch gekennzeichnet, daß** die Membran (11) der Membraneinheit (14) zwei aus einem nicht dehnbaren Material bestehende Membranlagen (11', 11") sowie einen sich zwischen beiden Membranlagen (11', 11") erstreckenden und mit einem inkompressiblen Medium derart gefüllten Zwischenraum (20) umfaßt, daß die Membranlagen (11', 11") eine bezüglich des Zwischenraumes (20) nach außen gerichtete Wölbung aufweisen.

## Claims

1. A pump device for delivering and metering in particular medical fluids comprising
a piston unit including a drive device (1) and a piston (7),
a diaphragm unit (14) which includes at least one diaphragm (11) and a first chamber (13) which is delimited by the diaphragm (11), and
a hydraulic unit (9) which has a space for accommodating a hydraulic fluid, which communicates with the piston (7) of the piston unit and the first chamber (13) of the diaphragm unit (14),
wherein there is provided a regulating unit for regulating a predeterminable metering rate and/or amount and a measuring device (8) by which the axial position of the piston (7) of the piston unit can be directly determined and which can be connected to the regulating unit,
**characterised in that** a head portion can be releasably connected to the diaphragm unit (14) in such a way that a second chamber arranged on the diaphragm side in opposite relationship to the first chamber (13) is formed, wherein the diaphragm pump head has at least one inlet and at least one outlet, and that the diaphragm layers (11', 11") are arranged separated from each other by a spacer portion (70).

2. A pump device according to claim 1 **characterised in that** the piston (7) has a piston head and a piston shaft and the measuring device (8) is arranged in such a way that the axial position of the piston shaft can be determined.

3. A pump device according to claim 1 or claim 2 **characterised in that** the measuring device (8) has optical and/or electronic sensors.

4. A pump device according to one of claims 1 to 3 **characterised in that** in the assembled condition of the diaphragm pump head the second chamber directly adjoins the diaphragm (11) of the diaphragm unit (14).

5. A pump device according to one of claims 1 to 3 **characterised in that** the second chamber is delimited by a diaphragm which in the assembled condition of the diaphragm pump head adjoins the diaphragm (11) of the diaphragm unit (14).

6. A pump device according to one of the preceding claims **characterised in that** the diaphragms bear against each other without clearance.

7. A pump device according to one or more of claims 1 to 5 **characterised in that** the inlet and/or the outlet of the diaphragm pump head can be closed off.

8. A pump device according to one or more of claims 1 to 7 **characterised in that** the head portion is such that it can be employed for disposable use.

9. A pump device according to one or more of claims 1 to 8 **characterised in that** there is provided a pressure pick-up (10) which communicates with the space of the hydraulic unit.

10. A pump device according to claim 9 **characterised in that** the pressure pick-up (10) can be connected by way of a computer or motor controller to the drive device (1) of the piston unit.

11. A pump device according to one or more of claims 1 to 10 **characterised in that** the drive device (1) of the piston unit includes a linear drive.

12. A pump device according to claim 11 **characterised in that** the linear drive is formed by a rack.

13. A pump device according to one or more of claims 1 to 12 **characterised in that** the hydraulic unit has a vent valve (12).

14. A pump device according to one or more of claims 1 to 13 **characterised in that** there is provided a computing unit which is connected to the measuring device (8) and/or to the regulating unit and by means of which metering, flow rate adjustment and therewith also balancing of the media being conveyed can be implemented.

15. A pump device according to claim 14 **characterised in that** the computing unit is integrated in the regulating unit.

16. A pump device according to one or more of claims 1 to 15 **characterised in that** the piston unit is arranged on a chassis (15).

17. A pump device according to one or more of claims 1 to 16 **characterised in that** the diaphragm (11) of the diaphragm unit (14) includes two diaphragm layers (11', 11") comprising a non-stretchable material and an intermediate space (20) which extends between the two diaphragm layers (11', 11") and which is filled with an incompressible medium in such a way that the diaphragm layers (11', 11") have a curvature directed outwardly with respect to the intermediate space (20).

## Revendications

1. Dispositif de pompage pour le convoyage et le dosage en particulier de liquides médicaux comportant
une unité à piston comprenant un dispositif d'entraînement (1) et un piston (7),
une unité à membrane (14) qui comprend au moins une membrane (11) ainsi qu'une première chambre (13) par laquelle la membrane (11) est délimitée, et
une unité hydraulique (9) qui présente un espace pour la réception d'un liquide hydraulique, qui est en liaison avec le piston (7) d'une unité à piston et la première chambre (13) de l'unité à membrane (14),
où est prévue une unité de réglage pour régler un taux et/ou quantité de dosage prédéfinissable ainsi qu'un dispositif de mesure (8), par lequel la position axiale du piston (7) de l'unité à piston peut être déterminée directement et qui peut être relié à l'unité de réglage,
**caractérisé**
**en ce qu'**avec l'unité à membrane (14), une pièce de tête peut être reliée relâchablement de façon qu'une deuxième chambre disposée au côté de la membrane opposé à la première chambre (13) soit formée, où la tête de la pompe à membrane présente au moins une entrée et au moins une sortie, et en ce que les couches de membrane (11', 11") sont disposées en étant séparées par une pièce d'écartement (70).

2. Dispositif de pompage selon la revendication 1, **caractérisé en ce que** le piston (7) présente une tête de piston et une tige de piston, et que le dispositif de mesure (8) est disposé de façon que la position axiale de la tige de piston peut être déterminée.

3. Dispositif de pompage selon la revendication 1 ou 2, **caractérisé en ce que** le dispositif de mesure (8) présente des capteurs optiques et/ou électroniques.

4. Dispositif de pompage selon l'une des revendications 1 à 3, **caractérisé en ce qu'**à l'état monté de la tête de la pompe à membrane, la deuxième chambre avoisine directement la membrane (11) de l'unité à membrane (14).

5. Dispositif de pompage selon l'une des revendications 1 à 3, **caractérisé en ce que** la deuxième chambre est délimitée par une membrane qui, à l'état monté de la tête de la pompe à membrane, avoisine la membrane (11) de l'unité à membrane (14).

6. Dispositif de pompage selon l'une des revendications précédentes, **caractérisé en ce que** les membranes reposent sans air l'une sur l'autre.

7. Dispositif de pompage selon l'une ou plusieurs des revendications 1 à 5, **caractérisé en ce que** l'entrée et/ou la sortie de la tête de la pompe à membrane peut être verrouillée.

8. Dispositif de pompage selon l'une ou plusieurs des revendications 1 à 7, **caractérisé en ce que** la pièce de tête est réalisée de façon qu'elle puisse être utilisée pour un usage unique.

9. Dispositif de pompage selon l'une ou plusieurs des revendications 1 à 8, **caractérisé en ce qu'**un capteur de pression (10) est prévu qui est en liaison avec l'espace de l'unité hydraulique.

10. Dispositif de pompage selon la revendication 9, **caractérisé en ce que** le capteur de pression (10) peut être relié par un calculateur ou un dispositif de commande moteur au dispositif d'entraînement (1) de l'unité à piston.

11. Dispositif de pompage selon l'une ou plusieurs des revendications 1 à 10, **caractérisé en ce que** le dispositif d'entraînement (1) de l'unité à piston comprend un dispositif d'entraînement linéaire.

12. Dispositif de pompage selon la revendication 11, **caractérisé en ce que** le dispositif d'entraînement linéaire est formé par une crémaillère.

13. Dispositif de pompage selon l'une ou plusieurs des revendications 1 à 12, **caractérisé en ce que** l'unité hydraulique présente une soupape d'aération (12).

14. Dispositif de pompage selon l'une ou plusieurs des revendications 1 à 13, **caractérisé en ce qu'**une unité de calcul est prévue qui est reliée au dispositif de mesure (8) et/ou à l'unité de réglage, et au moyen de laquelle peuvent être exécutés un dosage, un réglage du débit d'écoulement et donc également un établissement du bilan des milieux convoyés.

15. Dispositif de pompage selon la revendication 14, **caractérisé en ce que** l'unité de calcul est intégrée dans l'unité de réglage.

16. Dispositif de pompage selon l'une ou plusieurs des revendications 1 à 15, **caractérisé en ce que** l'unité à piston est disposée sur un châssis (15).

17. Dispositif de pompage selon l'une ou plusieurs des revendications 1 à 16, **caractérisé en ce que** la membrane (11) de l'unité à membrane (14) comprend deux couches de membrane (11', 11") en un matériau non extensible ainsi qu'un espace intermédiaire (20) s'étendant entre les deux couches de membrane (11', 11") et rempli d'un milieu non compressible de façon que les couches de membrane (11', 11") présentent un bombement dirigé relativement à l'espace intermédiaire (20) vers l'extérieur.
